# EUROPEAN PATENT APPLICATION

(11) **EP 4 775 129 A1**
(43) Date of publication of application: **15.07.2026**
(21) Application number: 24862447.0
(22) Date of filing: 19.07.2024
(51) Int. Cl.: A61B 3/06, G02B 5/20

(54) **COLOR VISION CHARACTERISTIC CORRECTION FILTER AND COLOR VISION EXAMINATION METHOD**

(30) Priority: 08.09.2023 JP 2023146574
(71) Applicant: Iris Communication Kabushiki Kaisha, Tokyo 101-0047 (JP); Nico Corporation, Tokyo 101-0047 (JP)
(72) Inventor: YANO Masafumi, Tokyo 101-0047 (JP)
(74) Representative: Lewis Silkin LLP
(86) International application number: PCT/JP2024/025887
(87) International publication number: WO 2025/052791

(57) **Abstract**

A correction filter is a correction filter configured to change a spectrum of transmitted light, and change a spectrum of light in a wavelength band including at least one of a peak absorption wavelength λL at which an absorption spectrum of L cone cells of a human peaks and a peak absorption wavelength λM at which an absorption spectrum of M cone cells of the human peaks. When changing a spectrum of light in a wavelength band including λL, transmittance increases as a wavelength goes from a wavelength XC between λL and λM toward a wavelength XL longer than λL. When changing a spectrum of light in a wavelength band including λM, transmittance increases as a wavelength goes from the wavelength XC toward a wavelength XS2 shorter than λM.

## Description

### BACKGROUND ART

The present invention relates to a correction filter for correcting a color vision characteristic and to a color vision testing method.

As disorders relating to human color vision, color blindness and color weakness, in which sensitivity to light in a specific wavelength band is low, and hypersensitiveness to light, in which light at a specific wavelength is perceived as glaring, are known. An example of the hypersensitiveness is Irlen syndrome. These color vision disorders occur because sensitivities of three types of cone cells (S cone cells, M cone cells, and L cone cells) and rod cells on a retina of a patient are higher or lower than those of a healthy individual. As a method for correcting color vision of the patient having a color vision disorder, a method is known that uses an optical filter whose light transmission characteristics are adjusted for the individual. When the patient wears glasses including the optical filter that characteristics have been adjusted, the color vision disorder of the patient is alleviated.

To produce an optical filter adjusted to a patient, it is necessary to test a color vision characteristic (i.e., sensitivity) of the patient with respect to light of various colors. However, since there are innumerable combinations of sensitivities for various lights, there has been a problem in that it is difficult to determine characteristics of an optical filter suitable for each patient.

A method for producing an optical filter tailored to a patient has been conventionally known. For example, Patent Document 1 (U.S. Patent Application Publication No. 2023/0016452) discloses a method for correcting a color vision characteristic of the patient by using an optical filter that greatly reduces transmittance of light in a yellow wavelength band that is between red and green wavelength bands.

[Patent Document 1] U.S. Patent Application Publication No. 2023/0016452

### SUMMARY

In the optical filter described in Patent Document 1, since transmittance in the yellow wavelength band is greatly reduced, even if a color vision characteristic of the patient is corrected, a problem may arise in that the patient becomes unable to recognize a color of a yellow object.

The present invention has been made in view of the above circumstances, and an object of the present invention is to provide a correction filter that correct a color vision characteristic suitable for each patient in accordance with the color vision characteristic of the patient and a color vision testing method, without greatly reducing intensity of light in a specific wavelength band.
correction filter according to an embodiment of the present invention is a correction filter configured to change a spectrum of transmitted light. The correction filter is configured to change a spectrum of light in a wavelength band including at least one of a peak absorption wavelength λL at which an absorption spectrum of L cone cells of a human peaks and a peak absorption wavelength λM at which an absorption spectrum of M cone cells of the human peaks.

With this configuration, a color vision disorder caused by a shift of a peak absorption wavelength at which an absorption spectrum of L cone cells or M cone cells of a human peaks can be corrected by changing a spectrum of light incident on a human eye. Further, the correction filter according to an embodiment of the present invention does not greatly reduce intensity of light in a specific wavelength band, and thus can prevent occurrence of a problem in which the patient becomes unable to recognize a specific color.

Further, the correction filter according to an embodiment of the present invention is set in such a manner that transmittance increases as a wavelength goes from a wavelength XC between the peak absorption wavelength λL and the peak absorption wavelength λM toward a wavelength XL longer than the peak absorption wavelength λL.

With this configuration, light in a red wavelength band among light incident on a human eye changes such that an intensity of the light becomes higher toward a longer-wavelength side, thereby it is possible to correct type-1 color vision caused by a peak absorption wavelength of the L cone cells shifting toward a shorter-wavelength side.

In addition, the correction filter according to an embodiment of the present invention is set in such manner that transmittance increases as a wavelength goes from a wavelength XC between the peak absorption wavelength λL and the peak absorption wavelength λM toward a wavelength XS2 shorter than the peak absorption wavelength λM.

With this configuration, light in a green wavelength band among the light incident on the human eye changes such that an intensity of the light becomes higher toward the shorter-wavelength side, thereby it is possible to correct type-2 color vision caused by a peak absorption wavelength of the M cone cells shifting toward the longer-wavelength side.

A color vision testing method according to an embodiment of the present invention is a color vision testing method using the correction filter. The color vision testing method includes a determination step of determining whether a particular condition is satisfied when a subject views a test image through the correction filter.

With this configuration, it is possible to test a color vision disorder caused by a peak absorption wavelength at which an absorption spectrum of the L cone cells or an absorption spectrum of the M cone cells peaks shifting.

According to an embodiment of the present invention, an optical filter suitable for each patient that corrects visual characteristics in accordance with visual characteristics of the patient, without significantly reducing an intensity of light in a specific wavelength band.

### BRIEF DESCRIPTION OF THE DRAWINGS

[FIG. 1] FIG. 1 shows absorption spectra of respective cone cells and rod cells of a human.
[FIG. 2] FIG. 2 shows exons included in a human red opsin gene and exons included in a human green opsin gene.
[FIG. 3] FIG. 3 shows a peak absorption wavelength of red opsin and a peak absorption wavelength of green opsin.
[FIG. 4] FIG. 4 shows relative absorption spectra of the L cone cells and the M cone cells of a human having normal color vision.
[FIG. 5] FIG. 5 shows relative absorption spectra of the L cone cells and the M cone cells of a human having mild type-1 color vision.
[FIG. 6] FIG. 6 shows relative absorption spectra of the L cone cells and the M cone cells of a human having mild type-2 color vision.
[FIG. 7] FIG. 7 shows relative absorption spectra of the L cone cells and the M cone cells of a human having mild type-1/type-2 mixed color vision.
[FIG. 8] FIG. 8 shows transmittance of a correction filter for correcting type-1 color vision.
[FIG. 9] FIG. 9 shows relative absorption spectra of the L cone cells and the M cone cells after correction in a human having mild type-1 color vision.
[FIG. 10] FIG. 10 shows transmittance of a correction filter for correcting type-1 color vision.
[FIG. 11] FIG. 11 shows transmittance of a correction filter for correcting type-1 color vision.
[FIG. 12] FIG. 12 shows transmittance of a correction filter for correcting type-1 color vision.
[FIG. 13] FIG. 13 shows transmittance of a correction filter for correcting type-2 color vision.
[FIG. 14] FIG. 14 shows relative absorption spectra of the L cone cells and the M cone cells after correction in a human having mild type-2 color vision.
[FIG. 15] FIG. 15 shows transmittance of a correction filter for correcting type-2 color vision.
[FIG. 16] FIG. 16 shows transmittance of a correction filter for correcting type-2 color vision.
[FIG. 17] FIG. 17 shows transmittance of a correction filter for correcting type-2 color vision.
[FIG. 18] FIG. 18 shows transmittance of a correction filter for correcting type-1/type-2 mixed color vision.
[FIG. 19] FIG. 19 shows relative absorption spectra of the L cone cells and the M cone cells after correction in a human having mild type-1/type-2 mixed color vision.
[FIG. 20] FIG. 20 shows transmittance of five correction filters for correcting type-1 color vision.
[FIG. 21] FIG. 21 shows transmittance of five correction filters for correcting type-1 color vision.
[FIG. 22] FIG. 22 shows transmittance of a correction filter for correcting type-1 color vision.
[FIG. 23] FIG. 23 shows transmittance of five correction filters for correcting type-2 color vision.
[FIG. 24] FIG. 24 shows transmittance of five correction filters for correcting type-2 color vision.
[FIG. 25] FIG. 25 shows transmittance of five correction filters for correcting type-1/type-2 mixed color vision.
[FIG. 26] FIG. 26 shows transmittance of five correction filters for correcting type-1/type-2 mixed color vision.

### DESCRIPTION

Hereinafter, an embodiment of the present invention will be described with reference to the drawings.

### (Human Color Vision Abnormality)

First, a mechanism by which a color vision abnormality of a human occurs will be described. Color vision characteristics of a human are determined based on characteristics of photoreceptor cells. The photoreceptor cells include three types of cone cells and one type of rod cells. The photoreceptor cells such as the cone cells and the rod cells are cells on a human retina. Because wavelength bands of light to which the photoreceptor cells have sensitivity differs depending on a type of the photoreceptor cells, the human can recognize light of various colors.

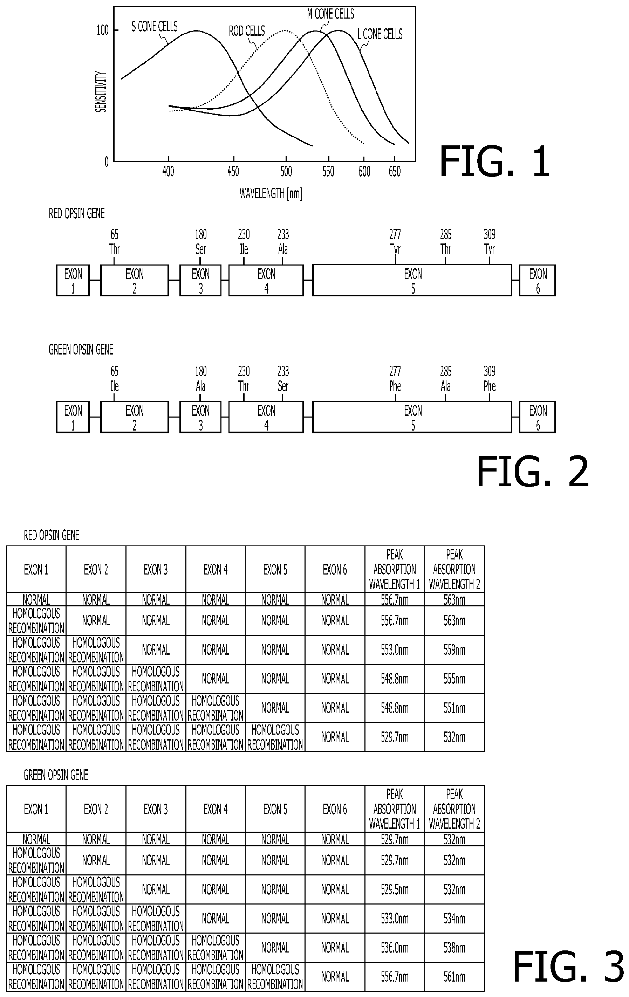

The cone cells include three types, namely, the S cone cells, the M cone cells, and the L cone cells. FIG. 1 shows an example of absorption spectra of the respective cone cells and the rod cells. The horizontal axis of FIG. 1 represents a wavelength of light, and the vertical axis represents absorption spectra of the respective cone cells and rod cells. Each of the absorption spectra shown in FIG. 1 is each normalized by a maximum value of an absorption rate. In the respective cone cells and rod cells, the higher the absorption rate is, the higher sensitivity to the light is. The S cone cells have maximum sensitivity around a wavelength of 420 nm. The M cone cells have maximum sensitivity around a wavelength of 534 nm. The L cone cells have maximum sensitivity around a wavelength of 563 nm. The rod cells have maximum sensitivity around a wavelength of 498 nm. Note that there are individual differences in a wavelength at which each of the cone cells and the rod cells has maximum sensitivity.

Some color vision disorders in humans are caused by the sensitivities of the cone cells and the rod cells being lower than, or higher than, those of a person with normal color vision. For example, a case in which the L cone cells have low sensitivity to red light is referred to as type-1 color vision, and a case in which the M cone cells have low sensitivity to green light is referred to as type-2 color vision. In the present embodiment, the color vision having characteristics of both the type-1 color vision and the type-2 color vision is referred to as type-1/type-2 mixed color vision.

Color vision abnormalities such as the type-1 color vision and the type-2 color vision are congenital color vision that is determined by whether a gene expresses a visual pigment involved in synthesis of the cone cells. The visual pigment is a complex of a visual pigment protein (i.e., opsin) and a vitamin A derivative (i.e., retinal). types of the opsin include the rhodopsin constituting the rod cells, and red opsin, green opsin, and blue opsin constituting the L cone cells, the M cone cells, and the S cone cells, respectively.

The rhodopsin and the blue opsin each consist of 348 amino acids. The gene encoding the rhodopsin (i.e., a rhodopsin gene) is present on the human chromosome 3, and the gene encoding the blue opsin (i.e., a blue opsin gene) is present on the human chromosome 7.

The red opsin and the green opsin each consist of 364 amino acids, and the gene encoding the red opsin (i.e., a red opsin gene) and the gene encoding the green opsin (i.e., a green opsin gene) encode proteins which are different in 15 amino acids out of the 364 amino acids. The red opsin gene and the green opsin gene are each present on Xq28 on the long arm of the human X chromosome, and normally, only the red opsin gene is expressed in the L cone cells and only the green opsin gene is expressed in the M cone cells.

Since the red opsin gene and the green opsin gene have similar gene sequences and are arranged adjacent to each other, homologous recombination between the red opsin gene and the green opsin gene may occur. When the homologous recombination occurs, abnormalities such as gene deletion in which a portion of an opsin gene is deleted, gene duplication in which a portion of a sequence of the red opsin gene and a portion of a sequence of the green opsin gene become partially the same, and formation of a hybrid gene including both a sequence of the red opsin gene and a sequence of the green opsin gene occur. A color vision abnormality occurs due to these abnormalities in the gene sequences.

For example, when a portion of the red opsin gene is replaced with a sequence of the green opsin gene by the homologous recombination, the resulting color vision becomes the type-1 color vision, and when a portion of the green opsin gene is replaced with a sequence of the red opsin gene, the resulting color vision becomes the type-2 color vision. In this case, a degree of the color vision abnormality depends on an extent to which replacement of the gene sequence has occurred.

FIG. 2 shows the exons included in the red opsin gene and the exons included in the green opsin gene. An exon is a region, in a gene sequence, that is involved in synthesis of an amino acid. The red opsin gene and the green opsin gene each include six exons from exon 1 to exon 6. Among the amino acids synthesized by these exons, the amino acids at positions 65, 180, 230, 233, 277, 285, and 309 are involved in the spectral absorption characteristics of an opsin, and in particular, the amino acids at positions 180, 277, and 285 are considered to have a large contribution to the spectral absorption characteristics. The amino acid at position 180 is an amino acid synthesized by exon 3, and the amino acids at positions 277 and 285 are amino acids synthesized by exon 5.

If no homologous recombination occurs in any exon and the red opsin and the green opsin are normally synthesized, the color vision of a human for red and green colors becomes normal. However, when the hybrid gene is formed due to homologous recombination of the genes, change of the spectral absorption characteristics occurs.

FIG. 3 shows a change in a wavelength at which an absorption rate of the red opsin peaks (i.e., a peak absorption wavelength) when the red opsin gene becomes a hybrid gene, and a change in a wavelength at which an absorption rate of the green opsin peaks (i.e., a peak absorption wavelength) when the green opsin gene becomes a hybrid gene. In FIG. 3, "Normal" indicates that the gene sequence is normal, and "Homologous recombination" indicates a case in which an exon of the red opsin gene is replaced with an exon of the green opsin gene, or a case in which an exon of the green opsin gene is replaced with an exon of the red opsin gene.

Of the two peak absorption wavelengths shown in FIG. 3, peak absorption wavelength 1 is based on a study by Merbs et al. (Merbs SL, et al.: Science (1992) 258: 464-466), and peak absorption wavelength 2 is based on a study by Asenjo et al. (Asenjo AB, et al.: Neuron (1994) 12: 1131-1138). Since the peak absorption wavelength has individual differences and measurement errors, a difference of several nm occurs between peak absorption wavelength 1 and peak absorption wavelength 2.

In a case where the homologous recombination does not occur in the red opsin gene, a maximum absorption wavelength of the red opsin is about 560 nm. In a case where the homologous recombination occurs in an exon of the red opsin gene and the exon sequence becomes an exon sequence of the green opsin gene, i.e., in a case of the type-1 color vision, the maximum absorption wavelength shifts from about 560 nm toward a shorter-wavelength side. The larger the number of exons in which the homologous recombination occurs, the larger an amount of shift of the maximum absorption wavelength becomes.

In a case where the homologous recombination does not occur in the green opsin gene, the maximum absorption wavelength of the green opsin is about 530 nm. In a case where the homologous recombination occurs in an exon of the green opsin gene and the exon sequence becomes an exon sequence of the red opsin gene, i.e., in a case of the type-2 color vision, the maximum absorption wavelength shifts from about 530 nm toward a longer-wavelength side. The larger the number of exons in which the homologous recombination occurs, the larger the amount of shift of the maximum absorption wavelength becomes.

The type-1/type-2 mixed color vision is a state in which both the homologous recombination of the red opsin gene and the homologous recombination of the green opsin gene occur, and thus both a shift of the maximum absorption wavelength of the red opsin and a shift of the maximum absorption wavelength of the green opsin occur.

In a mild type-1 color vision, the homologous recombination occurs in at most the exons 1 to 4, and in this case, the maximum absorption wavelength of the L cone cells shifts to about 550 nm. However, because there is a difference between the maximum absorption wavelength of the shifted L cone cells (about 550 nm) and about 530 nm which is the maximum absorption wavelength of the M cone cells, the type-1 color vision can be corrected (i.e., brought closer to the color vision characteristics of a person having normal color vision) by changing the spectrum of light incident on a human eye so as to correct the shift of the maximum absorption wavelength of the L cone cells. On the other hand, in a severe type-1 color vision, the homologous recombination occurs in exons 1 to 5 in the red opsin gene. In this case, the maximum absorption wavelength of the L cone cells shifts to about 530 nm, and the L cone cells have the highest sensitivity to green light. That is, in this state, the L cone cells and the M cone cells have substantially the same characteristics, and thus it becomes difficult to correct the severe type-1 color vision only by changing the spectrum of light incident on the human eye.

Similarly, in a mild type-2 color vision, the homologous recombination occurs in at most exons 1 to 4, and in this case, the maximum absorption wavelength of the green opsin shifts to about 535 nm. However, because there is a difference between the maximum absorption wavelength of the shifted M cone cells (about 535 nm) and about 560 nm which is the maximum absorption wavelength of the L cone cells, the type-2 color vision can be corrected (i.e., brought closer to the color vision characteristics of the person having normal color vision) by changing the spectrum of light incident on the human eye so as to correct the shift of the maximum absorption wavelength of the M cone cells. On the other hand, in a severe type-2 color vision, the homologous recombination occurs in exons 1 to 5 in the green opsin gene. In this case, the maximum absorption wavelength of the M cone cells shifts to about 560 nm, and the M cone cells have the highest sensitivity to red light. That is, in this state, the M cone cells and the L cone cells have substantially the same characteristics, and thus it becomes difficult to correct the severe type-2 color vision only by changing the spectrum of light incident on the human eye.

Next, color vision characteristics of a human will be described. The L cone cells and the M cone cells of the human output a signal corresponding to an intensity of received light. According to a hypothesis by Stell et al. (Stell WK, Lightfoot DO, Wheeler TG, Leeper HF (1975) Goldfish retina: Functional polarization of cone horizontal cell dendrites and synapses. Science 190: 989-990), an output from the L cone cells is input to H-type horizontal cells and is then input to the M cone cells. The M cone cells output, to R/G-type horizontal cells, a signal corresponding to the intensity of the received light and signals input from L-type horizontal cells. An output from the R/G-type horizontal cells corresponds to a difference between the output from the L cone cells and the output from the M cone cells, in other words, corresponds to an output corresponding to a difference between an intensity of red light and an intensity of green light. Then, outputs from the H-type horizontal cells and the R/G-type horizontal cells are transmitted to a visual cortex via retinal ganglion cells (e.g., amacrine cells, bipolar cells, etc.), whereby a color vision is obtained.

That is, according to the hypothesis by Stell et al., it is inferred that red and green color are not recognized such that outputs of the L cone cells and the M cone cells directly correspond thereto, but are recognized based on the output of the L cone cells and a difference between the output of the L cone cells and the output of the M cone cells. Therefore, in a case where an intensity of light is corrected by the correction filter, it is inferred that the color vision abnormality can be corrected by correcting an intensity of red light to which the L cone cells have sensitivity and a difference between the intensity of the red light and an intensity of green light to which the M cone cells have sensitivity.

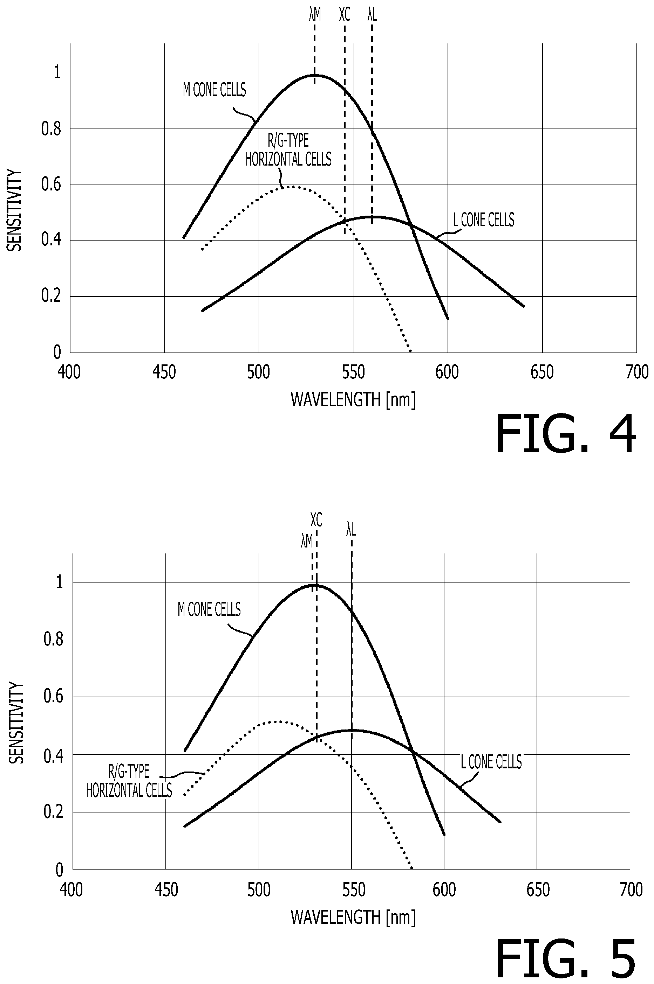

FIG. 4 illustrates relative absorption spectra of the L cone cells and the M cone cells of a person having normal color vision. In FIG. 1, each absorption spectrum of each cone cells is normalized by a maximum value; however, in FIG. 4, the relative absorption spectrum of the L cone cells is shown with the maximum value of the absorption spectrum of the M cone cells used as a reference. FIG. 4 also shows a difference between the absorption spectrum of the M cone cells and the absorption spectrum of the L cone cells (which is obtained by subtracting the absorption spectrum of the L cone cells from the absorption spectrum of the M cone cells). The difference corresponds to the output from the R/G-type horizontal cells.

A person generally recognizes light of a single wavelength of about 546 nm as green, and recognizes light of a single wavelength of about 580 nm as yellow. The relative absorption spectra of the L cone cells and the M cone cells shown in FIG. 4 are estimated on an assumption that a wavelength of green light is about 546 nm and a wavelength of yellow light is about 580 nm. Green light and red light become yellow light when mixed at equal intensities. Therefore, it is inferred that, at the wavelength of yellow light (e.g., about 580 nm), the sensitivity of the L cone cells and the sensitivity of the M cone cells become equal. Further, a person recognizes green light based on the output from the R/G-type horizontal cells corresponding to a difference between the intensity of red light and the intensity of green light. Therefore, it is inferred that, at the wavelength of green light (e.g., 546 nm), the intensity of red light relative to the output from the R/G-type horizontal cells becomes zero, i.e., the sensitivity of the M cone cells becomes twice the sensitivity of the L cone cells. The relative absorption spectra of the L cone cells and the M cone cells shown in FIG. 4 are based on these inferences.

Further, according to the hypothesis by Stell et al., a person recognizes red light in accordance with the output from the L cone cells and recognizes green light in accordance with the output from the R/G-type horizontal cells. Accordingly, in FIG. 4, it is inferred that light on a longer-wavelength side than near the wavelength XC (e.g., about 546 nm in a person having normal color vision) at which the output from the R/G-type horizontal cells (which is obtained by subtracting the absorption spectrum of the L cone cells from the absorption spectrum of the M cone cells) intersects the absorption spectrum of the L cone cells is light recognized as a reddish color, and light on a shorter-wavelength side than the wavelength XC is light recognized as a greenish color.

Fig. 5 shows relative absorption spectra of the L cone cells and the M cone cells of a person having mild type-1 color vision. The horizontal axis in Fig. 5 indicates the wavelength of the light, and the vertical axis indicates the absorption spectra of the L cone cells and the M cone cells. Fig. 5 also shows a difference between the absorption spectrum of the M cone cells and the absorption spectrum of the L cone cells (i.e., a spectrum obtained by subtracting the absorption spectrum of the L cone cells from the absorption spectrum of the M cone cells). Fig. 5 shows an example in which exons 1 to 4 of the red opsin gene are respectively replaced with exons 1 to 4 of the green opsin gene, and a maximum absorption wavelength of the L cone cells shifts from about 560 nm to a shorter wavelength side to become about 550 nm.

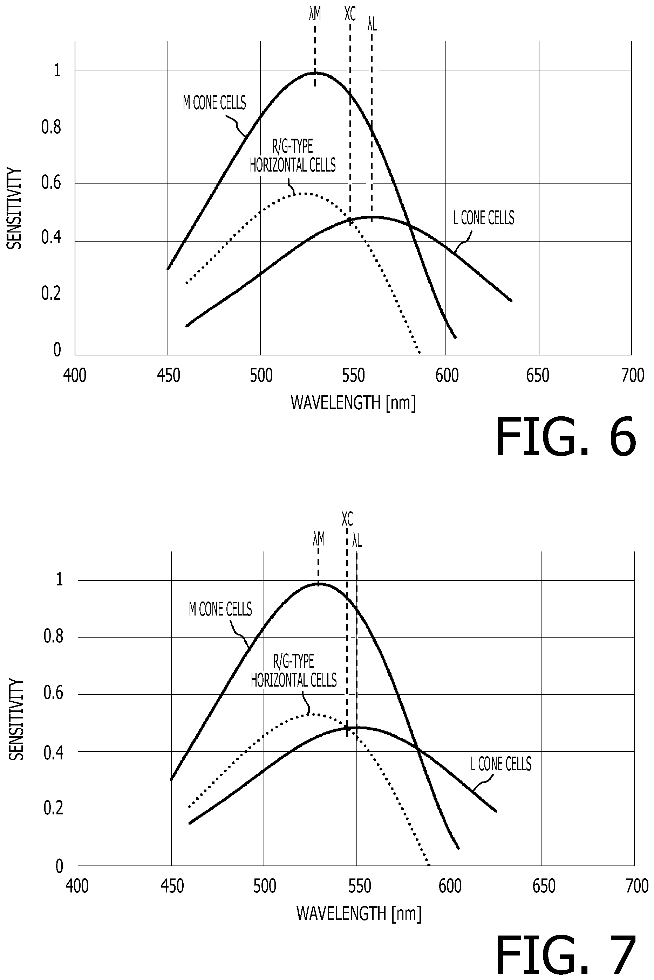

Fig. 6 shows relative absorption spectra of the L cone cells and the M cone cells of a person having mild type-2 color vision. The horizontal axis in Fig. 6 indicates the wavelength of the light, and the vertical axis indicates the absorption spectra of the L cone cells and the M cone cells. Fig. 6 also shows a difference between the absorption spectrum of the M cone cells and the absorption spectrum of the L cone cells (i.e., a spectrum obtained by subtracting the absorption spectrum of the L cone cells from the absorption spectrum of the M cone cells). Fig. 6 shows an example in which exons 1 to 4 of the green opsin gene are respectively replaced with exons 1 to 4 of the red opsin gene, and a maximum absorption wavelength of the M cone cells shifts from about 530 nm to a longer wavelength side to become about 535 nm.

Fig. 7 shows relative absorption spectra of the L cone cells and the M cone cells of a person having both mild type-1 color vision and mild type-2 color vision, i.e., type-1/type-2 mixed color vision. The horizontal axis in Fig. 7 indicates the wavelength of the light, and the vertical axis indicates the absorption spectra of the L cone cells and the M cone cells. Fig. 7 also shows a difference between the absorption spectrum of the M cone cells and the absorption spectrum of the L cone cells (i.e., a spectrum obtained by subtracting the absorption spectrum of the L cone cells from the absorption spectrum of the M cone cells). In the type-1/type-2 mixed color vision, since the person has characteristics of both the type-1 color vision and the type-2 color vision, the maximum absorption wavelength of the L cone cells becomes about 550 nm, and the maximum absorption wavelength of the M cone cells becomes about 535 nm.

The type-1 color vision, the type-2 color vision, or the type-1/type-2 mixed color vision can be corrected by changing a spectrum of light incident on the human eye such that the absorption spectra of the L cone cells and the M cone cells shown in Figs. 6 to 7 are brought closer to the absorption spectra of the L cone cells and the M cone cells of a normal person shown in Fig. 5. In other words, the color vision can be corrected by matching the shifted maximum absorption wavelength to the maximum absorption wavelength of the normal person and matching relative magnitudes of sensitivities of the L cone cells and the M cone cells to magnitudes of sensitivities of the normal person.

The correction filter according to the present embodiment is configured, based on color vision characteristics of a subject such as the type-1 color vision, the type-2 color vision, or the type-1/type-2 mixed color vision, to perform correction for bringing the shifted maximum absorption wavelength of the L cone cells or the M cone cells closer to the maximum absorption wavelength of the normal person, and to perform correction for adjusting an intensity ratio between red light and green light.

### (Correction Filter)

Next, the correction filter for correcting mild type-1 color vision, mild type-2 color vision, or type-1/type-2 mixed color vision will be described. The correction filter corrects the color vision of a subject, and is assumed to be worn on eyes of the subject. For example, the correction filter may be worn by the subject like a pair of glasses or contact lenses. Alternatively, the correction filter may be attached to a television, a monitor screen, or the like used by the subject, thereby adjusting colors of the television or the monitor screen.

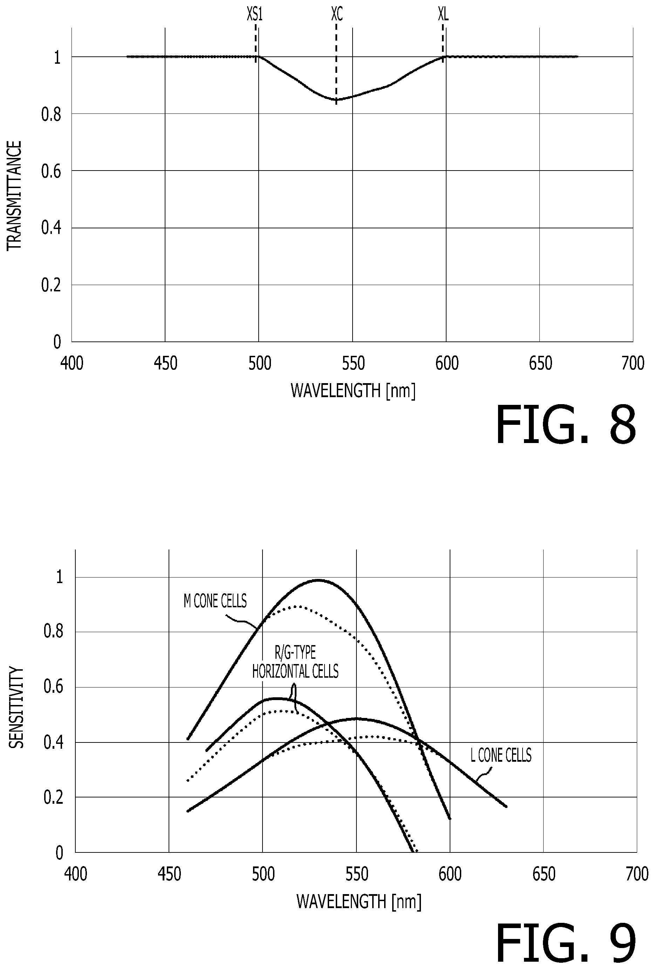

Fig. 8 shows transmittance of the correction filter for correcting the type-1 color vision. The horizontal axis in Fig. 8 indicates the wavelength of the light, and the vertical axis indicates the transmittance of the correction filter. The correction filter shown in Fig. 8 is a filter that brings the maximum absorption wavelength of the L cone cells in the type-1 color vision closer to the maximum absorption wavelength of the L cone cells in the normal person, and adjusts a difference or a ratio between an intensity of red light and an intensity of green light.

The correction filter shown in Fig. 8 is set in such a manner that, between the maximum absorption wavelength λM of the M cone cells in the type-1 color vision and the maximum absorption wavelength λL of the L cone cells, the transmittance increases as the wavelength moves from the wavelength XC, at which an output from the R/G-type horizontal cells and the absorption spectrum of the L cone cells intersect each other, toward the wavelength XL that is longer than the maximum absorption wavelength λL of the L cone cells.

Since the transmittance of the correction filter is inclined to increase as the wavelength goes from the wavelength XC toward the wavelength XL, an intensity, of the light transmitted through the correction filter, around the maximum absorption wavelength λL of the L cone cells becomes lower, and an intensity of the light on a longer-wavelength side than the wavelength λL becomes relatively higher. Therefore, when the subject having the type-1 color vision wears the correction filter, a peak of the absorption spectrum of the L cone cells of the subject shifts to the longer-wavelength side. Accordingly, in the type-1 color vision, the peak of sensitivity of the L cone cells that is shifted to the shorter-wavelength side can be brought closer to the peak of sensitivity of the L cone cells of the normal person.

Note that the wavelength XL may be set to a wavelength that a human recognizes as yellow with respect to light having the single wavelength XL. The wavelength XL is, for example, about 580 nm. This reduces a decrease in the intensity of yellow light caused by the correction filter, thereby suppressing a change in a color vision of the human with respect to yellow light due to the correction filter.

In addition, in the correction filter shown in FIG. 8, the transmittance is set to increase as the wavelength goes from the wavelength XC toward the wavelength XS 1, which is shorter than the maximum absorption wavelength λM of the M cone cells. This causes the intensity of the green light transmitted through the correction filter to decrease. This is because the transmittance of the green light is reduced in accordance with the transmittance of light having wavelengths between the wavelength XC and the wavelength XL (i.e., the red light) being reduced, thereby balancing the intensity of the red light and the intensity of the green light.

The wavelength XS1 may be set to the maximum absorption wavelength (e.g., 498 nm) of the rod cells. This can suppress a decrease in the intensity of light received by the rod cells due to the correction filter.

### (Correction of Type-1 Color Vision)

FIG. 9 shows corrected absorption spectra of the L cone cells and the M cone cells when a subject having the type-1 color vision shown in FIG. 5 wears the correction filter shown in FIG. 8. The horizontal axis of FIG. 9 indicates a wavelength of light, and the vertical axis indicates the absorption spectra of the L cone cells and the M cone cells. Further, a difference between an absorption spectrum of the M cone cells and an absorption spectrum of the L cone cells, which corresponds to an output of the R/G-type horizontal cells, is also shown. In FIG. 9, solid lines indicate the absorption spectra before the correction filter is worn, and dotted lines indicate the absorption spectra corrected by the correction filter. The corrected absorption spectrum is obtained by multiplying the absorption spectra of the L cone cells and the M cone cells by the transmittance of the correction filter of FIG. 8 at each wavelength.

In the type-1 color vision, the maximum absorption wavelength of the L cone cells is shifted from about 560 nm in a person having normal color vision to about 550 nm. In contrast, the correction filter operates to return the maximum absorption wavelength of the L cone cells to about 560 nm by changing the spectrum of light incident on the human eye. Thus, the characteristics of the L cone cells in the type-1 color vision can be corrected. In addition, the correction filter decreases the intensity of light in a green wavelength band transmitted therethrough. Thus, when correcting the characteristics of the L cone cells, it is possible to correct a balance between the intensity of the red light and the intensity of the green light that has changed due to the intensity of the red light being decreased.

The correction filter shown in FIG. 8 is set such that the transmittance increases as the wavelength goes from the wavelength XC toward the wavelength XL, and is also set such that the transmittance increases as the wavelength goes from the wavelength XC toward a shorter-wavelength side. As a result, the transmittance is set to have a minimum value in the vicinity of the wavelength XC. However, in the present embodiment, the correction filter for correcting the type-1 color vision is not limited to this setting.

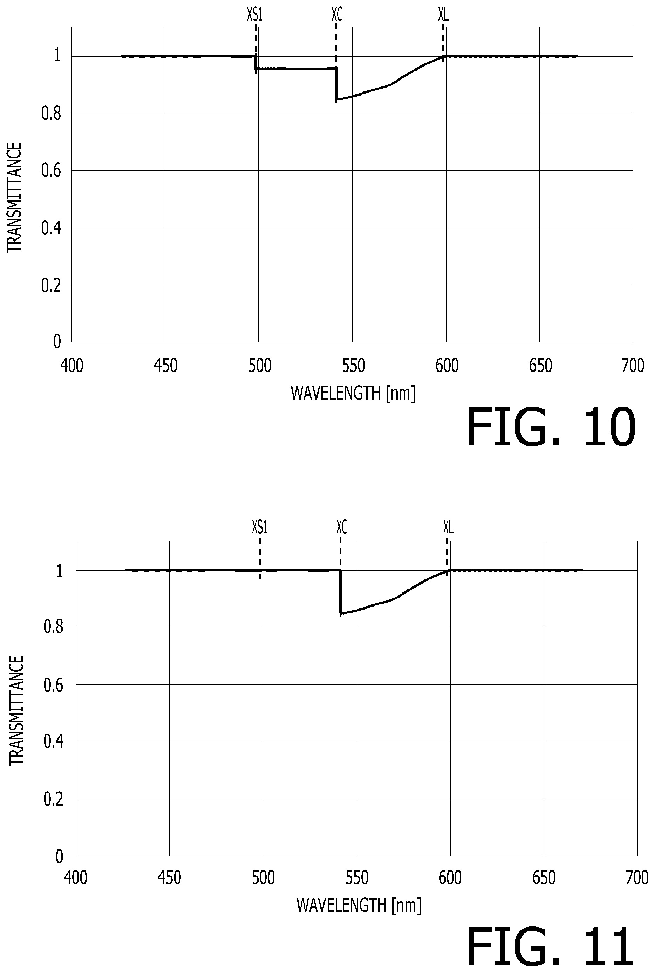

In the present embodiment, for example as shown in FIG. 10, the transmittance may be constant in a wavelength band from the wavelength XC to the wavelength XS1, which is shorter than the maximum absorption wavelength λM of the M cone cells. Even with this configuration, it is possible to adjust a difference or a ratio between the intensity of the red light and the intensity of the green light.

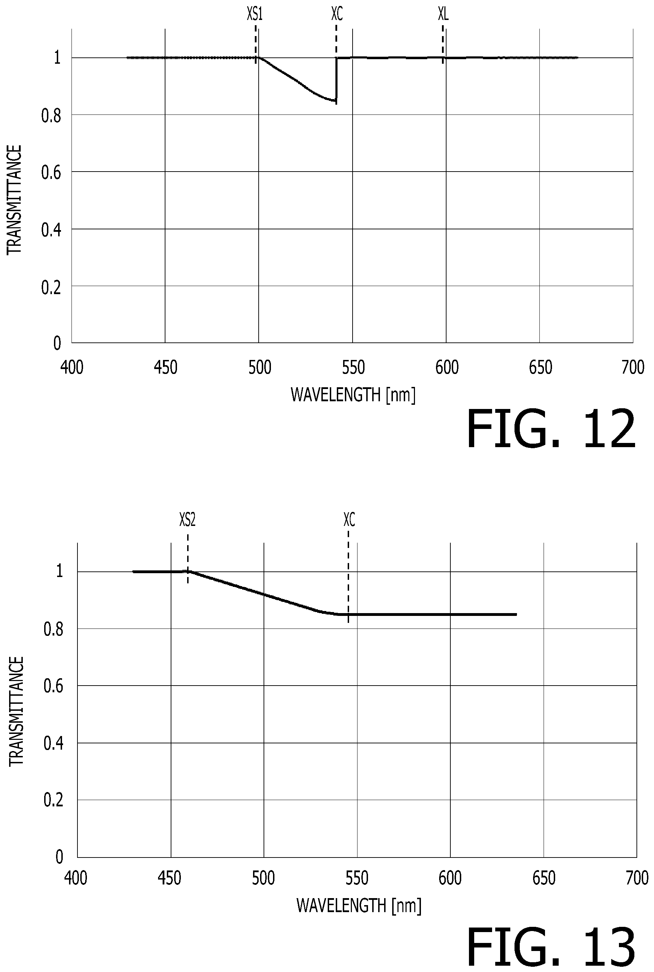

Further, the correction filter does not need to be a single filter. For example, two correction filters shown in FIGS. 11 and 12 may be combined. The correction filter shown in FIG. 11 is set such that the transmittance increases as the wavelength goes from the wavelength XC toward the wavelength XL, and the transmittance of the correction filter is set to be high (e.g., about 100%) in a wavelength band from the wavelength XC to the wavelength XS1. In the correction filter shown in FIG. 12, the transmittance is set such that the transmittance increases as the wavelength goes from the wavelength XC toward the wavelength XS1, and the transmittance of the correction filter is set to be high (e.g., about 100%) in a wavelength band from the wavelength XC to the wavelength XL. By combining the two filters in this manner, it is possible to adjust a difference or a ratio between the intensity of the red light and the intensity of the green light, and also to correct the type-1 color vision.

FIG. 13 shows transmittance of an example of the correction filter for correcting the type-2 color vision. The horizontal axis of FIG. 13 indicates a wavelength of light, and the vertical axis indicates the transmittance of the correction filter. The correction filter shown in FIG. 13 is a filter that brings the maximum absorption wavelength of the M cone cells in the type-2 color vision closer to the maximum absorption wavelength of the M cone cells in a person having normal color vision.

The correction filter shown in FIG. 13 is set such that, between the maximum absorption wavelength λM of the M cone cells in the type-2 color vision and the maximum absorption wavelength λL of the L cone cells, the transmittance increases as the wavelength goes from the wavelength XC, at which the output from the R/G-type horizontal cells and the absorption spectrum of the L cone cells intersect each other, toward the wavelength XS2 that is longer than the maximum absorption wavelength λM of the M cone cells.

Because the transmittance of the correction filter is inclined to increase as the wavelength goes from the wavelength XC toward the wavelength XM, among the light transmitted through the correction filter, the intensity in the vicinity of the maximum absorption wavelength λM of the M cone cells becomes low, and the intensity of light on a shorter-wavelength side than the wavelength λM becomes relatively high. Therefore, when a subject having the type-2 color vision wears the correction filter, the peak of the absorption spectrum of the M cone cells of the subject acts to shift toward a shorter-wavelength side. Thus, in the type-2 color vision, it is possible to bring the peak of the sensitivity of the M cone cells, which is shifted to a longer-wavelength side, closer to the peak of the sensitivity of the M cone cells of a person having normal color vision.

In addition, in the correction filter shown in FIG. 13, the transmittance is set to decrease on a longer-wavelength side than the wavelength XC. This causes the intensity of the red light transmitted through the correction filter to decrease. This is because the transmittance of light having wavelengths between the wavelength XC and the wavelength XS2 (i.e., the green light) is decreased in the correction filter, and the transmittance of the red light is decreased accordingly, thereby balancing the intensity of the red light and the intensity of the green light.

Note that the wavelength XS2 may be set to a wavelength that a human recognizes as blue with respect to light having a single wavelength at the wavelength XS2. The wavelength XS2 is, for example, about 450 nm. This reduces a decrease in the intensity of the blue light due to the correction filter, thereby suppressing a change in a human's color vision with respect to the blue light due to the correction filter.

### (Correction of Type-2 Color Vision)

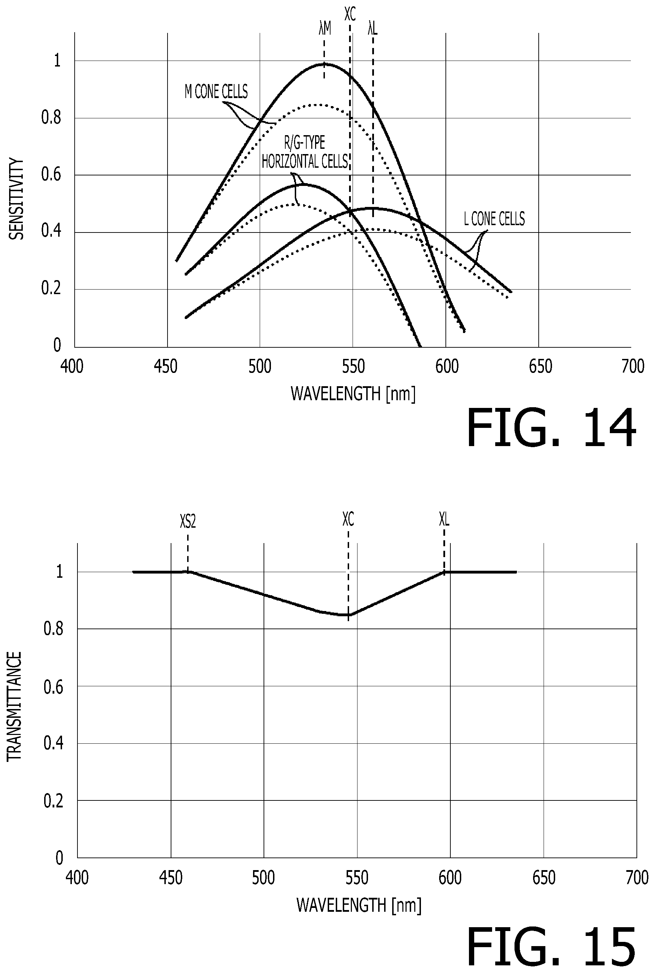

FIG. 14 shows the corrected absorption spectra of the L cone cells and the M cone cells when the subject having the type-2 color vision shown in FIG. 6 wears the correction filter shown in FIG. 13. The horizontal axis of FIG. 14 indicates a wavelength of light, and the vertical axis indicates the absorption spectra of the L cone cells and the M cone cells. In addition, a difference between the absorption spectrum of the M cone cells and the absorption spectrum of the L cone cells, which corresponds to the output of the R/G-type horizontal cells, is also shown. In FIG. 14, solid lines indicate the absorption spectra before the correction filter is worn, and dotted lines indicate the absorption spectra corrected by the correction filter. The corrected absorption spectra are obtained by multiplying the absorption spectra of the L cone cells and the M cone cells by the transmittance of the correction filter of FIG. 13 at each wavelength.

In the type-2 color vision, the peak absorption wavelength of the M cone cells is shifted from about 530 nm in a person having normal color vision to about 535 nm. In contrast, the correction filter operates to return the peak absorption wavelength of the M cone cells to around about 530 nm by changing the spectrum of light incident on a human eye. Thus, the characteristics of the M cone cells in the type-2 color vision can be corrected. In addition, the correction filter decreases the intensity of light in a red wavelength band transmitted therethrough. Thus, when correcting the characteristics of the M cone cells, it is possible to correct the intensity balance between the red light and the green light that has changed due to the intensity of the green light being decreased.

In the correction filter shown in FIG. 13, the transmittance is set to be constant on a longer-wavelength side than the wavelength XC. However, in the present embodiment, the correction filter for correcting the type-2 color vision is not limited to this setting. For example, as shown in FIG. 15, the transmittance may be set to increase as the wavelength goes from the wavelength XC toward the wavelength XL that is longer than the peak absorption wavelength λL of the L cone cells. Even with this configuration, it is possible to adjust a difference or a ratio between the intensity of the red light and the intensity of the green light.

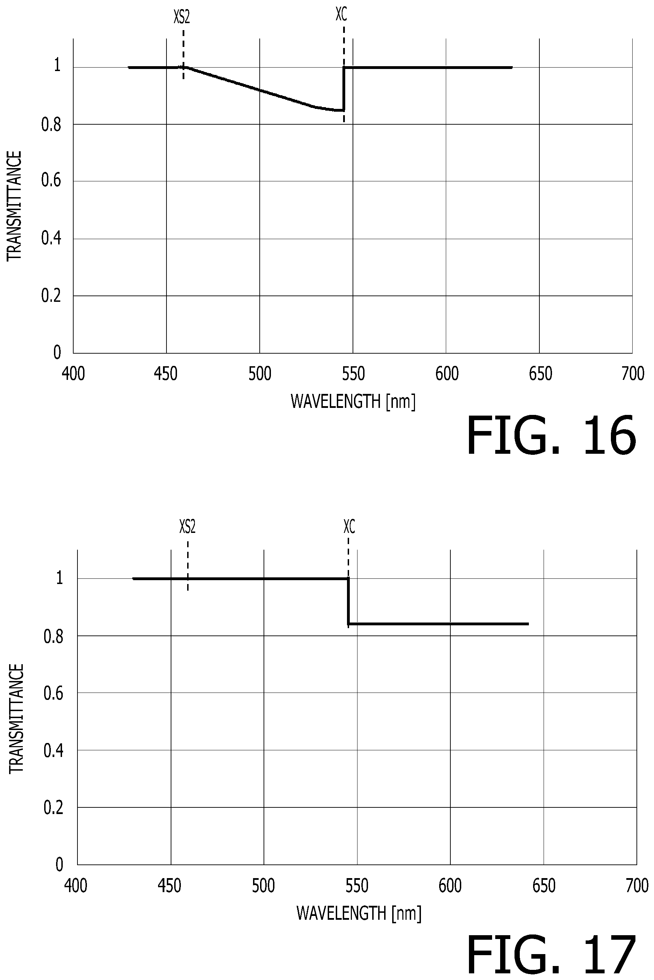

In addition, the correction filter does not need to be a single filter. For example, two correction filters shown in FIGS. 16 and 17 may be combined. In the correction filter shown in FIG. 16, the transmittance is set to increase as the wavelength goes from the wavelength XC toward the wavelength XS2, and the transmittance in other wavelength bands is set to be high (e.g., approximately 100%). In the correction filter shown in FIG. 17, the transmittance in a wavelength band on a longer-wavelength side than the wavelength XC is set to be lower than the transmittance in other wavelength bands. By combining the two filters in this manner, it is possible to adjust a difference or a ratio between the intensity of the red light and the intensity of the green light, and also to correct the type-2 color vision.

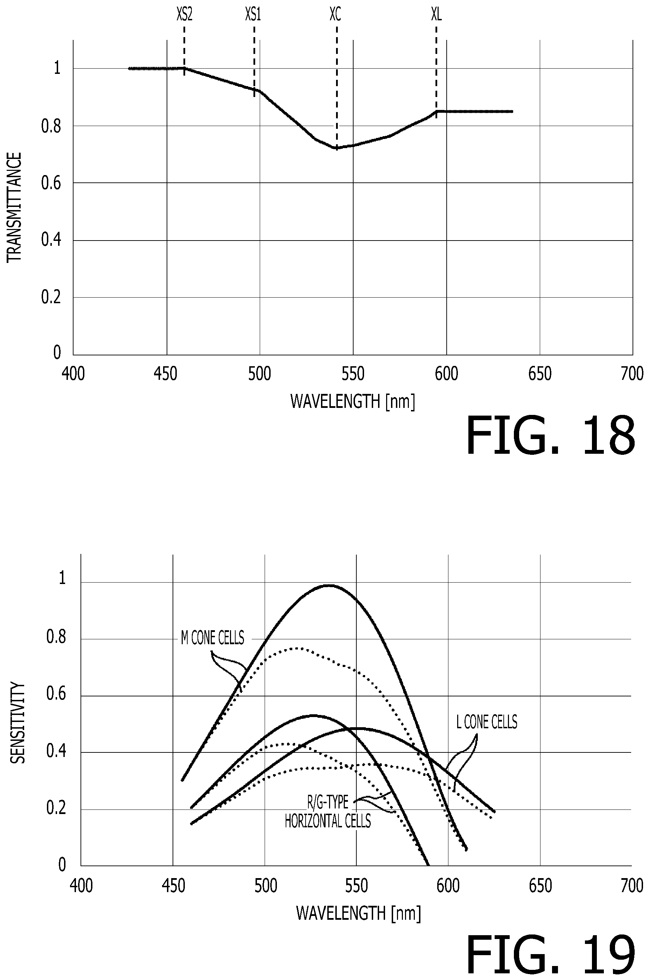

FIG. 18 shows the transmittance of an example of the correction filter for correcting the type-1/type-2 mixed color vision. The horizontal axis of FIG. 18 indicates a wavelength of light, and the vertical axis indicates the transmittance of the correction filter. The correction filter shown in FIG. 18 has characteristics obtained by combining the correction filter for correcting the type-1 color vision and the correction filter for correcting the type-2 color vision.

The correction filter shown in FIG. 18 is set such that, between the peak absorption wavelength λM of the M cone cells and the peak absorption wavelength λL of the L cone cells in the type-1/type-2 mixed color vision, the transmittance increases as the wavelength goes from the wavelength XC, at which the output from the R/G-type horizontal cells and the absorption spectrum of the L cone cells intersect, toward the wavelength XL that is longer than the peak absorption wavelength λL of the L cone cells.

Because the transmittance of the correction filter is inclined to increase as the wavelength goes from the wavelength XC toward the wavelength XL, when the subject having the type-1/type-2 mixed color vision wears the correction filter, the peak of the absorption spectrum of the L cone cells of the subject shifts toward a longer-wavelength side. Thus, in the type-1/type-2 mixed color vision, it is possible to bring the peak of the sensitivity of the L cone cells, which is shifted toward a shorter-wavelength side, closer to the peak of the sensitivity of the L cone cells of a person having normal color vision.

In addition, the correction filter shown in FIG. 18 is set such that, from the wavelength XC between the peak absorption wavelength λM of the M cone cells and the peak absorption wavelength λL of the L cone cells in the type-1/type-2 mixed color vision, the transmittance increases as the wavelength goes toward the wavelength XS2 that is shorter than the peak absorption wavelength λM of the M cone cells.

Because the transmittance of the correction filter is inclined to increase as a wavelength goes from the wavelength XC toward the wavelength XM, when the subject having the type-1/type-2 mixed color vision wears the correction filter, the peak of the absorption spectrum of the M cone cells of the subject shifts toward a shorter-wavelength side. Thus, in the type-2 color vision, it is possible to bring the peak of the sensitivity of the M cone cells, which is shifted toward a longer-wavelength side, closer to the peak of the sensitivity of the M cone cells of a person having normal color vision.

### (Correction of Type-1/Type-2 Mixed Color Vision)

FIG. 19 shows corrected absorption spectra of the L cone cells and the M cone cells when the subject having the type-1/type-2 mixed color vision shown in FIG. 7 wears the correction filter shown in FIG. 18. In addition, a difference between the absorption spectrum of the M cone cells and the absorption spectrum of the L cone cells, which corresponds to the output of the R/G-type horizontal cells, is also shown. The horizontal axis of FIG. 19 indicates a wavelength of light, and the vertical axis indicates the absorption spectra of the L cone cells and the M cone cells. In FIG. 19, solid lines indicate the absorption spectrum before the correction filter is worn, and dotted lines indicate the absorption spectrum corrected by the correction filter. The corrected absorption spectrum is obtained by multiplying the absorption spectra of the L cone cells and the M cone cells by the transmittance of the correction filter of FIG. 18 at each wavelength.

In the type-1/type-2 mixed color vision, the peak absorption wavelength of the L cone cells is shifted from about 560 nm in a person having normal color vision to about 550 nm, and the peak absorption wavelength of the M cone cells is shifted from about 530 nm in the person having normal color vision to about 535 nm. In contrast, the correction filter operates to return the peak absorption wavelength of the L cone cells to around about 560 nm and to return the peak absorption wavelength of the M cone cells to around about 530 nm, by changing the spectrum of light incident on a human eye. Thus, it is possible to correct the characteristics of the M cone cells in the type-1/type-2 mixed color vision.

Note that the correction filter for correcting the type-1/type-2 mixed color vision does not need to be constituted by a single filter. For example, the correction filter for correcting the type-1/type-2 mixed color vision may be constituted by superposing the correction filter for correcting the type-1 color vision and the correction filter for correcting the type-2 color vision.

### (Color Vision Testing Method)

In the present embodiment, the correction filter is used to correct the color vision of the subject having the type-1 color vision, the type-2 color vision, the type-1/type-2 mixed color vision, or the like, however, the present embodiment is not limited to this configuration. For example, the correction filters may be used to test the color vision of the subject.

When performing a color vision test, the subject wears the correction filter for correcting the type-1 color vision, the type-2 color vision, or the type-1/type-2 mixed color vision, and observes the surrounding scenery and a test image. The test image may be, for example, a known Ishihara color vision test image. The Ishihara color vision test image is an image generally used in a test for a color vision abnormality. The Ishihara color vision test image is colored such that a figure (for example, characters such as numerals) can be recognized when the color vision is normal, or when the color vision abnormality is appropriately corrected. Since a test using the Ishihara color vision test image is well known to a person skilled in the art, an explanation thereof is omitted in the present specification.

The subject observes the test image through the correction filter while changing the type of the correction filter to be worn and the degree of correction. The type of the correction filter includes a filter for correcting the type-1 color vision, a filter for correcting the type-2 color vision, a filter for correcting the type-1/type-2 mixed color vision, and the like. In addition, the degree of correction is changed by changing the transmittance of the correction filter. By observing the test image while changing the correction filter to be worn by the subject and selecting the correction filter with which the subject can clearly recognize a figure included in the test image, it is possible to test the color vision characteristics of the subject. In addition, the correction filter with which the subject can clearly recognize the figure included in the test image can be used as a filter for correcting the color vision characteristics of the subject. The condition that the subject can clearly recognize the figure included in the test image is an example of a particular condition of the present application.

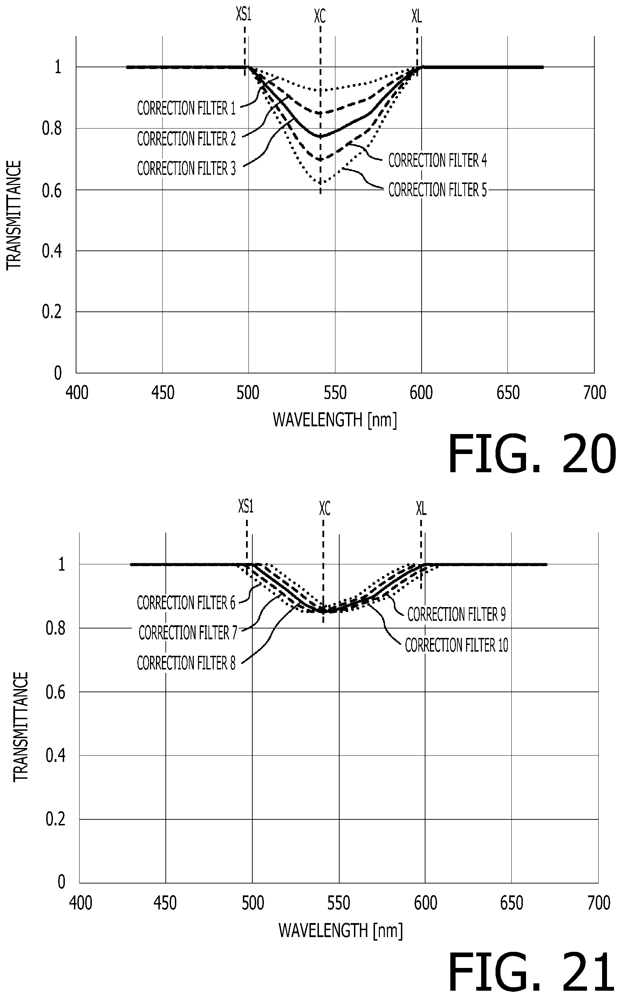

FIG. 20 shows the transmittance of five correction filters, namely the correction filter 1 to the correction filter 5, for correcting the type-1 color vision. The horizontal axis of FIG. 20 indicates a wavelength of light, and the vertical axis indicates the transmittance of the correction filters. The plurality of the correction filters 1 to 5 shown in FIG. 20 have the same wavelength XC at which the transmittance becomes a minimum, and the same wavelength (i.e., the wavelength XS1 and the wavelength XL) at which the transmittance begins to decrease toward the wavelength XC, namely, the same wavelength band in which the transmittance is low, but have transmittances that are different from each other.

Since there are individual differences in the color vision characteristics of a human, for example, even if humans have the same type-1 color vision, the sensitivity to the red light and the sensitivity to the green light are different depending on the human. The difference in the sensitivity is caused by, for example, the number of the L cone cells, the number of the M cone cells, and a ratio therebetween. Therefore, as shown in FIG. 20, by using a plurality of correction filters having different transmittances, it is possible to test the color vision characteristics of the subject more accurately.

FIG. 21 shows the transmittance of five correction filters, namely the correction filter 6 to the correction filter 9, for correcting the type-1 color vision. The horizontal axis of FIG. 21 indicates a wavelength of light, and the vertical axis indicates the transmittance of the correction filters. The plurality of the correction filters 6 to 10 shown in FIG. 21 are different from each other in the wavelength XC at which the transmittance becomes a minimum and the wavelength (i.e., the wavelength XS1 and the wavelength XL) at which the transmittance begins to decrease toward the wavelength XC, namely, the wavelength band in which the transmittance is low.

As described above, the peak absorption wavelength of the M cone cells of a human shifts depending on how many homologous recombinations of exons have occurred in the green opsin gene, and the peak absorption wavelength of the L cone cells shifts depending on how many homologous recombinations of exons have occurred in the red opsin gene. Therefore, as shown in FIG. 21, by using a plurality of correction filters having different wavelength regions in which the transmittance is low, it is possible to test the color vision characteristics of the subject more accurately.

Note that the correction filters 1 to 5 shown in FIG. 20 are different from each other in the transmittance at the wavelength XC in increments of about 5%, however, the configuration of the present embodiment is not limited thereto. In order to test the color vision characteristics of the subject more accurately, the plurality of correction filters may are different from each other in the transmittance at the wavelength XC in smaller increments (for example, in increments of about 3%). Alternatively, in order to perform the test more quickly, the plurality of correction filters may are different from each other in larger increments (for example, in increments of about 10%).

Further, the correction filters 6 to 7 shown in FIG. 21 are different from each other in the wavelength band in which the transmittance is low in increments of about 5 nm, however, the configuration of the present embodiment is not limited thereto. In order to test the color vision characteristics of the subject more accurately, the plurality of correction filters may are different from each other in the wavelength band in which the transmittance is low in smaller increments (for example, in increments of about 3 nm). Alternatively, in order to perform the test more quickly, the plurality of correction filters may are different from each other in the wavelength band in which the transmittance is low in larger increments (for example, in increments of about 10 nm).

Further, FIG. 20 shows characteristics of the plurality of correction filters 1 to 5 in which the wavelength band in which the transmittance is low is not changed and only the transmittance is different from each other, and FIG. 21 shows characteristics of the plurality of correction filters 6 to 9 in which the transmittance is the same but the wavelength band in which the transmittance is low is different from each other; however, the characteristics of the correction filter of the present embodiment are not limited thereto. A plurality of correction filters in which both the transmittance and the wavelength band in which the transmittance is low are different from each other may be used for testing or correcting the color vision characteristics of the subject.

Further, in the correction filters shown in FIG. 20 and FIG. 21, the transmittance is set to 1 (approximately 100%) on a shorter-wavelength side than the wavelength XS1 and on a longer-wavelength side than the wavelength XL, however, the correction filter of the present embodiment is not limited to this configuration. The transmittance on the shorter-wavelength side than the wavelength XS1, or on the longer-wavelength side than the wavelength XL, may be a value smaller than 1. Thus, since the intensity of light in the visible light band incident on the eye of the subject decreases, for example, it becomes easier to test the color vision characteristics of the subject having a photosensitivity disorder in which the subject feels light as dazzling. Further, the correction filter in which the transmittance on a shorter-wavelength side than the wavelength XS1, or on a longer-wavelength side than the wavelength XL, is set to a value smaller than 1 may be used to correct the color vision characteristics of the subject having the photosensitivity disorder in which the subject feels light as dazzling.

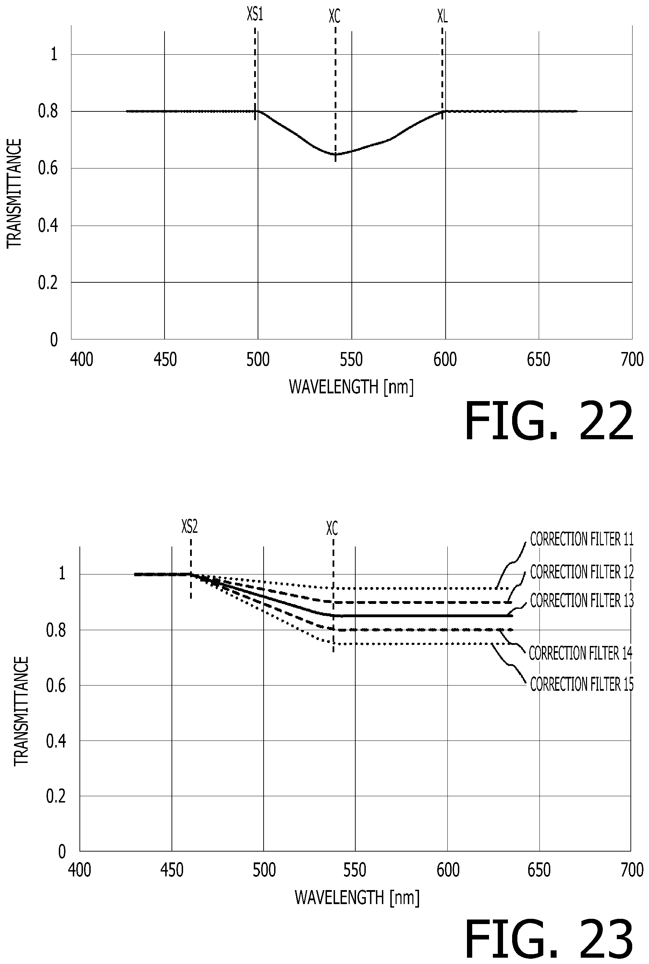

FIG. 22 is a diagram showing the transmittance of the correction filter according to another embodiment. The horizontal axis of FIG. 22 indicates a wavelength of light, and the vertical axis indicates the transmittance of the correction filter. The correction filter shown in FIG. 22 is obtained by offsetting the correction filter shown in FIG. 8 such that the transmittance becomes low over the entire visible light band. In a range from the wavelength XS1 to the wavelength XL, the transmittance is set to be lower than the transmittance on a shorter-wavelength side than the wavelength XS1 and on a longer-wavelength side than the wavelength XL. In this manner, by decreasing the transmittance over the entire visible light band, for example, it becomes easier to test the color vision characteristics of the subject having the photosensitivity disorder in which the subject feels light as dazzling. The offset of the transmittance is not limited to the example shown in FIG. 22; for example, the color vision characteristics of the subject may be tested using a plurality of correction filters having different offset amounts. Further, the correction filter whose transmittance is offset may be used to correct the color vision characteristics of the subject having the photosensitivity disorder in which the subject feels light as dazzling.

Note that FIG. 20 and FIG. 21 show characteristics of the correction filters for testing or correcting the color vision characteristics of the subject having the type-1 color vision, however, the present embodiment is not limited to this configuration. By changing the transmittance of the correction filter and the region in which the transmittance becomes low, it is possible to test or correct the color vision characteristics of the subject having the type-2 color vision or the type-1/type-2 mixed color vision.

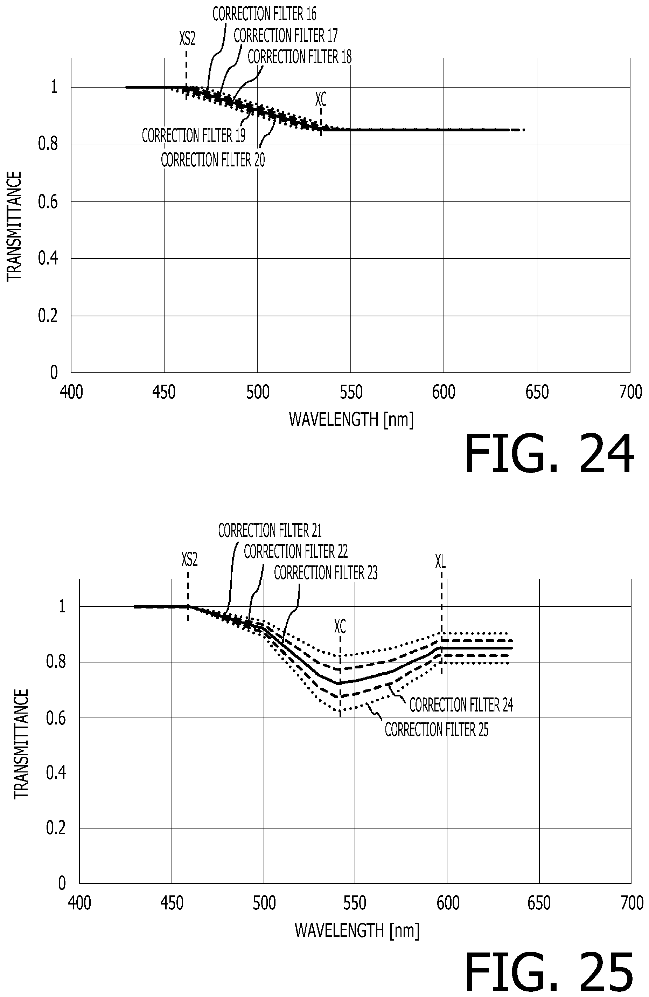

FIG. 23 and FIG. 24 show characteristics of the correction filters for testing the color vision characteristics of the subject having the type-2 color vision. The horizontal axes of FIG. 23 and FIG. 24 indicate wavelengths of light, and the vertical axes indicate the transmittances of the correction filters. In the plurality of correction filters 11 to 15 shown in FIG. 23, the wavelengths XC at which the transmittance becomes a minimum and the wavelength XS2 at which the transmittance begins to decrease toward the wavelength XC (i.e., the wavelength band in which the transmittance is low) are the same, but the transmittances are different from each other. In the plurality of correction filters 16 to 20 shown in FIG. 24, the wavelengths XC at which the transmittance becomes a minimum and the wavelengths XS2 at which the transmittance begins to decrease toward the wavelength XC (i.e., the wavelength band in which the transmittance is low) are different from each other.

The subject observes the test image while changing the correction filters 11 to 20 to be worn, and, by selecting the correction filter with which the characters of the test image are most easily recognized, it is possible to test the characteristics of the type-2 color vision of the subject. Further, the correction filter with which the subject can clearly recognize the figure included in the test image can be used as the filter for correcting the color vision characteristics of the subject.

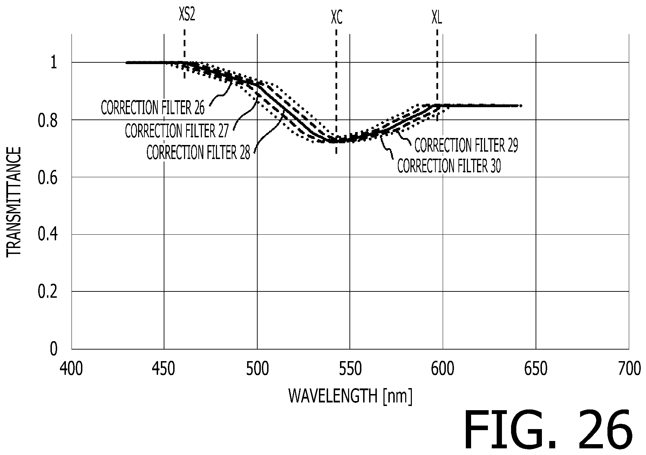

FIG. 25 and FIG. 26 show characteristics of the correction filters for testing the color vision characteristics of the subject having the type-1/type-2 mixed color vision. The horizontal axes of FIG. 25 and FIG. 26 indicate wavelengths of light, and the vertical axes indicate the transmittances of the correction filters. In the plurality of correction filters 21 to 25 shown in FIG. 25, the wavelengths XC at which the transmittance becomes a minimum and the wavelength (i.e., the wavelength XS2 and the wavelength XL) at which the transmittance begins to decrease toward the wavelength XC, namely, the wavelength band in which the transmittance is low, are the same, but the transmittances are different from each other. In the plurality of correction filters 26 to 30 shown in FIG. 26, the wavelengths XC at which the transmittance becomes a minimum and the wavelength (i.e., the wavelength XS2 and the wavelength XL) at which the transmittance begins to decrease toward the wavelength XC, namely, the wavelength band in which the transmittance is low, are different from each other.

The subject observes the test image while changing the correction filters 21 to 30 to be worn, and, by selecting the correction filter with which the characters of the test image are most easily recognized, it is possible to test the characteristics of the type-1/type-2 mixed color vision of the subject. In addition, the correction filter with which the subject can clearly recognize the figure included in the test image can be used as the filter for correcting the color vision characteristics of the subject.

Note that the correction filters 11 to 15 shown in FIG. 23 and the correction filters 21 to 25 shown in FIG. 25 are different from each other in the transmittance at the wavelength XC in increments of about 5%. However, the configuration of the present embodiment is not limited this configuration. In order to test the color vision characteristics of the subject more accurately, the plurality of correction filters may be different from each other in the transmittance at the wavelength XC in smaller increments (for example, in increments of about 3%). Alternatively, in order to perform the test more quickly, the plurality of correction filters may be different from each other in larger increments (for example, in increments of about 10%).

Further, in the correction filters shown in FIG. 23 and FIG. 24, the transmittance is set to 1 (approximately 100%) on a shorter-wavelength side than the wavelength XS2. However, the correction filter of the present embodiment is not limited to this configuration. The transmittance on the shorter-wavelength side than the wavelength XS2 may be a value smaller than 1. In addition, the correction filters shown in FIG. 23 and FIG. 24 may have the transmittance uniformly offset such that the transmittance decreases over the entire visible light band.

Further, the correction filters 16 to 20 shown in FIG. 24 and the correction filters 26 to 30 shown in FIG. 25 are different from each other in the wavelength band in which the transmittance is low in increments of about 5 nm. However, the configuration of the present embodiment is not limited thereto. In order to test the color vision characteristics of the subject more accurately, the plurality of correction filters may be different from each other in the wavelength band in which the transmittance is low in smaller increments (for example, in increments of about 3 nm). Alternatively, in order to perform the test more quickly, the plurality of correction filters may be different from each other in larger increments (for example, in increments of about 10 nm).

Further, in the correction filters shown in FIG. 25 and FIG. 26, the transmittance is set to 1 (approximately 100%) on a shorter-wavelength side than the wavelength XS2 and on a longer-wavelength side than the wavelength XL; however, the correction filter of the present embodiment is not limited to this configuration. The transmittance on the shorter-wavelength side than the wavelength XS2, or on the longer-wavelength side than the wavelength XL, may be a value smaller than 1. In addition, the correction filters shown in FIG. 25 and FIG. 26 may have the transmittance uniformly offset such that the transmittance decreases over the entire visible light band.

Further, the correction filters 11 to 30 differ from each other in either the transmittance or the wavelength band in which the transmittance is low; however, the configuration of the present embodiment is not limited thereto. A plurality of correction filters in which both the transmittance and the wavelength band in which the transmittance is low differ from each other may be used for testing or correcting the color vision characteristics of the subject.

In this manner, by testing the color vision characteristics of the subject using the plurality of correction filters, it is possible to produce the correction filter for correcting the color vision characteristics of the subject based on the test result.

In the present embodiment, testing and correction of the type-1 color vision, the type-2 color vision, and the type-1/type-2 mixed color vision have been described. However, an embodiment of the present invention is not limited to this configuration. For example, when the peak absorption wavelength at which the absorption spectrum of the S cone cells, the rod cells, or the like of the subject peaks is different from that of a person having normal color vision, testing and correction of the color vision of the subject may be performed using the correction filter that shifts the peak absorption wavelength.

The foregoing is a description of exemplary embodiments of the present invention. An embodiment of the present invention is not limited to the embodiment described above, and various modifications are possible within the scope of the technical idea of the present invention. For example, contents obtained by appropriately combining embodiments exemplarily disclosed in the present specification and/or obvious embodiments are also included in an embodiment of the present invention.

## Claims

1. A correction filter configured to change a spectrum of transmitted light,
wherein the correction filter is configured to change a spectrum of light in a wavelength band including at least one of a peak absorption wavelength λL at which an absorption spectrum of L cone cells of a human peaks and a peak absorption wavelength λM at which an absorption spectrum of M cone cells of the human peaks.

2. The correction filter according to claim 1,
wherein the correction filter is set in such a manner that transmittance increases as a wavelength goes from a wavelength XC between the peak absorption wavelength λL and the peak absorption wavelength λM toward a wavelength XL longer than the peak absorption wavelength λL.

3. The correction filter according to claim 2,
wherein the wavelength XC is set in a vicinity of a wavelength at which a difference spectrum obtained by subtracting the absorption spectrum of the L cone cells from the absorption spectrum of the M cone cells intersects with the absorption spectrum of the the L cone cells.

4. The correction filter according to claim 3,
wherein the wavelength XC is about 540 nm.

5. The correction filter according to any one of claims 2 to 4,
wherein the wavelength XL is a wavelength that a human recognizes as yellow with respect to monochromatic light having the wavelength XL.

6. The correction filter according to claim 5,
wherein the wavelength XL is about 580 nm.

7. The correction filter according to any one of claims 2 to 6,
wherein the correction filter is a filter configured to correct a color vision characteristic of a subject, and
wherein the correction filter is configured to change an intensity of light in a wavelength band from the wavelength XC to the wavelength XL based on the color vision characteristic of the subject.

8. The correction filter according to any one of claims 2 to 7,
wherein the correction filter is set in such a manner that transmittance increases as a wavelength goes from the wavelength XC toward a wavelength XS1 shorter than the peak absorption wavelength λM.

9. The correction filter according to claim 8,
wherein the wavelength XS1 is set in a vicinity of a peak absorption wavelength λROD at which an absorption spectrum of L cone cells of the human peaks.

10. The correction filter according to claim 8,
wherein the wavelength XS1 is about 498 nm.

11. The correction filter according to claim 1,
wherein the correction filter is set in such manner that transmittance increases as a wavelength goes from a wavelength XC between the peak absorption wavelength λL and the peak absorption wavelength λM toward a wavelength XS2 shorter than the peak absorption wavelength λM.

12. The correction filter according to claim 11,
wherein the wavelength XC is set in a vicinity of a wavelength at which a difference spectrum obtained by subtracting the absorption spectrum of the L cone cells from the absorption spectrum of the M cone cells intersects with the absorption spectrum of the L cone cells.

13. The correction filter according to claim 12,
wherein the wavelength XC is about 546 nm.

14. The correction filter according to any one of claims 11 to 13,
wherein the wavelength XS2 is set in a vicinity of a wavelength at which the absorption spectrum of the M cone cells intersects with an absorption spectrum of S cone cells of the human.

15. The correction filter according to any one of claims 11 to 13,
wherein the wavelength XS2 is about 450 nm.

16. The correction filter according to any one of claims 11 to 15,
wherein the correction filter is a filter configured to correct a color vision characteristic of a subject, and
wherein the correction filter is configured to change an intensity of light in a wavelength band from the wavelength XC to the wavelength XS2 based on the color vision characteristic of the subject.

17. The correction filter according to claim 1,
wherein the correction filter is set in such manner that transmittance increases as a wavelength goes from a wavelength XC between the peak absorption wavelength λL and the peak absorption wavelength λM toward a wavelength XL longer than the peak absorption wavelength λL, and
wherein the correction filter is set in such manner that transmittance increases as a wavelength goes from the wavelength XC toward a wavelength XS2 shorter than the peak absorption wavelength λM.

18. A correction filter comprising a correction filter according to any one of claims 1 to 10 and a correction filter according to any one of claims 11 to 16, the correction filters being superposed on each other.

19. A color vision testing method using a correction filter according to any one of claims 1 to 18,
wherein the color vision testing method comprising a determination step of determining whether a particular condition is satisfied when a subject views a test image through the correction filter.
